# EUROPEAN PATENT SPECIFICATION

(11) **EP 3 338 573 B1**
(45) Date of publication and mention of the grant of the patent: **11.10.2023**
(21) Application number: 16867504.9
(22) Date of filing: 10.03.2016
(51) Int. Cl.: A41D 13/12, A61N 1/04, A61N 1/36, A41D 1/00, A61B 5/25

(54) **METHOD OF MANUFACTURING TRAINING GARMENTS FOR USE IN EMS**
VERFAHREN ZUR HERSTELLUNG VON TRAININGSBEKLEIDUNG FÜR DEN EMS-EINSATZ
PROCEDE DE FABRICATION DE VETEMENTS D'ENTRAINEMENT A UTILISER EN EMS

(30) Priority: 23.11.2015 CN 201510815900
(43) Date of publication of application: 27.06.2018
(73) Proprietor: Zhejiang Revformula Health Tech Co., Ltd, Ningbo, Zhejiang 315171 (CN)
(72) Inventor: DONG, Qing, Ningbo Zhejiang 315171 (CN)
(74) Representative: Murgitroyd & Company
(86) International application number: PCT/CN2016/000124
(87) International publication number: WO 2017/088275

(56) References cited:
- WO-A1-2014/090927
- CN-A- 101 081 323
- CN-A- 104 510 466
- CN-U- 202 665 528
- CN-U- 203 408 044
- CN-U- 204 207 739
- DE-U1-202014 103 548
- FR-A1- 2 613 594
- JP-A- 2000 316 988
- KR-B1- 100 781 319
- US-A1- 2002 077 688
- US-A1- 2002 077 689
- US-A1- 2012 245 483
- US-A1- 2013 072 777
- US-A1- 2013 085 538
- LU , XIN.: "The Tailoring Process of Male Jacket", Clothes Sewing Technology and Management, vol. 1, no. 1, 28 February 2005 (2005-02-28), pages 305-309, XP009510921, ISBN: 7-5064-3246-3

## Description

### Field of the Invention

The present invention relates to a garment manufacturing process, and in particular to a method for manufacturing training garments for use in Electrical Muscle Stimulation (EMS).

### Description of the Prior Art

The metabolism and all activities throughout life will generate electricity. External stimuli, heart beating, muscular contraction, eyes opening/closing, brain thinking and the like are accompanied by the generation and transformation of bioelectricity. Physiological studies indicate that the bioelectricity is not the by-product or accompaniment of an organ or functional activities of the organ, but the key or decisive factor for cells to realize some important physiological functions. Particularly, electromyographic signals transmitted by nerve cells stimulate muscles to make responses, so that the muscular system applies an acting force to the skeletal system to perform various mechanical actions.

Bioelectrical signals transmitted by a human body, such as heartbeats (electrocardiographic signals) and muscular contraction (electromyographic signals), are collected by electrodes, filtered, amplified and then analyzed.

Electricity may also be applied to a human body from the outside via electrodes. High-voltage electropulse applications such as defibrillators and low-voltage electropulse applications such as EMS can be used to stimulate muscles so that the muscles make a contraction response to achieve the effect of training muscles.

In the EMS, as the frequency of the current, an intermediate frequency and a low frequency are included; and electrodes are pairwise disposed around a muscle group. The surface and deep muscles and the fast-twitch and slow-twitch muscles can be all simultaneously "mobilized" to participate in activities by electrical signals. Moreover, by wave packets at a certain frequency, the muscles can be contracted and relaxed quickly, so that the effect of training muscles which is far beyond that achievable by relying on the nervous system of the human body can be achieved. With the development of the EMS, it has been quickly expanded to people's daily exercises in addition to the field of professional training. From the use experience of ordinary persons, a wearable electrode device capable of covering the whole human body is urgently demanded, in which electrodes and electric wires are both fitted to the human body, and the current generator is small and portable and can be controlled by wireless Bluetooth. The electrodes can be fully fitted to the outline of the human body. The current is distributed uniformly without any local peak discharge which stimulates the human body, and the electric wires connecting the current generator with the electrodes are highly elastic and thus make the user feel comfortable, so that the free movement of the human body is ensured.

With the development of material, wearable devices have been designed for the EMS intelligent micro-electricity technology. For example, Chinese Patent Publication No. CN101081323A (Application No. 200610084275.3) has disclosed a wearable electrode device and a manufacturing method thereof, wherein the manufacturing method comprises the following steps of: weaving a piece of cloth, wherein the cloth comprises N separate conducting cloth blocks; according to a pattern of a garment, tailoring a semi-finished product of the garment from this piece of cloth, the semi-finished product comprising M conducting regions formed by the N conducting cloth blocks; in each of the M conducting regions of this semi-finished product, providing an individual electrical connection component, for connecting the M conducting regions with an external device; and, producing the garment from the semi-finished product having the electrical connection components. Although the wearable electrode device manufactured by this manufacturing method have various forms, the steps required to produce the finished garment are complicated, and the correspondence of the conducting regions to muscle groups, the level of comfort of the finished product, the degree of combination with the EMS features and the like are still to be improved.

In addition, there is also a method for manufacturing a wearable electrode device, wherein electrodes are additionally provided on a finished garment. For example, Chinese Patent Publication No. CN101536903A (Application No. 200910064618.3) has disclosed a wearable electrocardio electrode device and a manufacturing method thereof, wherein the manufacturing method comprises the following steps of: tailoring a garment according to the pattern of the garment; using composite fiber as a substrate, applying a nano-silver layer onto the composite fiber to form silver fiber, and providing the manufactured electrodes at corresponding positions of the garment; and, providing leads in the garment to connect the electrodes to an external device. In this manufacturing method, as the way of fixing the electrodes onto the garment, it is possible to sew the electrodes onto the garment by threads, or adhere the electrodes onto the garment by glue, or adhere the electrodes onto the garment by hook and loop fasteners. However, if by sewing, since the garment is three-dimensional and the sewing operation needs to be performed manually instead of being automatically performed by a machine because the existing sewing machines, embroidery machines and the like can only operate on a two-dimensional object, it is highly labor-consuming; and if by using glue or the hook and loop fasteners, the level of comfort when a person wearing the garment will be influenced, and the stability of fixation is somewhat low.

Document US2002077688 discloses a garment which may cover a portion of the body or the entire body for quickly and accurately positioning a plurality of electrode pads at select muscle groups and/or nerve areas for providing optimal and efficient muscle and nerve electrical stimulation treatment.

### Summary of the Invention

It is an object of the present invention to provide a method for manufacturing training garments for use in Electrical Muscle Stimulation (EMS), which has accurate electrode alignment and shortened manufacturing time.

For achieving this object, there is provided a method for manufacturing training garments for use in EMS as claimed in claim 1.

Optional further features of the method are defined in the dependent claims.

The method comprises:
1) preparing a semi-finished training garment, comprising:
   (A) fabric tailoring, comprising: tailoring a piece of elastic fabric to a plane cloth piece and lower sleeve pieces, the plane cloth piece having: a front chest piece (32), a first back piece (33) and a second back piece (34), the front chest piece located in a middle portion of the plane cloth piece and the first and second back pieces located on respective left and right sides of the front chest piece; a first leg piece (35) located on the left side of the front chest piece and below the first back piece, and a second leg piece (36) located on the right side of the front chest piece and below the second back piece; a collar piece (37) located above the front chest piece; and shoulder pieces (38) that are located on respective sides of the collar piece;
   (B) fixing close-fitting electrodes, comprising: fixing a plurality of close-fitting electrodes onto a back side of the cloth piece and lower sleeve pieces by an embroidery machine, in which the close-fitting electrodes, in use, directly face and are fitted at positions corresponding to muscle groups of a human body, wherein each of the close-fitting electrodes comprises a piece of silver-plated cloth, a thin film, and an elastic layer, which are are compounded in turn;
   (C) fixing elastic electric leads, comprising: fixing elastic electric leads onto the back side of the cloth piece and lower sleeve pieces by the embroidery machine, so as to connect symmetrical close-fitting electrodes together;
   (D) sewing waist darts, comprising: synchronously threading at positions of the cloth piece corresponding to the waistline positions of the human body to define regions for sewing waist darts, and then fixing and reinforcing by sewing densely and zigzagging;
   (E) providing an electric generator connector, comprising: providing an electric connector on a front side of the cloth piece, the electric connector being used for connecting to an external EMS electric generator, the electric connector being electrically connected to all the elastic electric leads on the back side of the cloth piece; and
2) manufacturing a finished training garment, comprising:
   folding left and right portions of the cloth piece obtained in the step (E), partially sewing the folded cloth piece at a junction of inner sides of each of the leg pieces and at a junction of the first and second back pieces, providing a slide fastener in the remaining portion of the junction of the back pieces so as to form a garment body, sewing the lower sleeve pieces to positions of the garment body corresponding to the arms to form sleeves, and connecting the external EMS electric generator to the electric connector to form a training garment for use in EMS.

Preferably,. Adhering the thin film onto the silver-plated cloth can cause the electric conductivity to be uniform, sweat to be quickly absorbed, and the loss of silver to be reduced. Accordingly, the resistivity can be decreased, and the washing durability can be improved. By compounding the elastic layer and the base cloth, the close fitting of the electrodes to the human body can be ensured during the motion of the human body, and the electrode cloth can be protected as a whole.

The close-fitting electrodes are manufactured in a method comprising the following steps:
(i) manufacturing the silver-plated cloth, comprising: using nylon fiber as a substrate to obtain the silver-plated cloth;
(ii) adhering the thin film, comprising: adhering the thin film onto the silver-plated cloth;
(iii) compounding the elastic layer, comprising: compounding the elastic layer on the thin film to obtain the compounded electrode cloth; and
(iv) cutting, comprising: cutting the electrode cloth according to the desired size to obtain the close-fitting electrodes.

In step (iii) of manufacturing the close-fitting electrodes, an elastic fabric layer is further compounded on the elastic layer of the electrode cloth to form aprotective base cloth. The base cloth can reduce the noise caused by the friction of the electrodes with the skin of the human body.

In the present invention, preferably, the elastic electric leads are formed by twisting and merging a plurality of silver-plated nylon fiber filaments and polyurethane. Such electric leads are suitable for sports, good in elasticity, comfortable in fitting effect and low in energy consumption, thus can satisfy the requirements on the battery life of a portable device, and still have low loss of silver and good conductivity after multiple times of washing.

Preferably, in the step of fixing the elastic electric leads, the elastic electric leads are drawn, by a crochet hook, into triangular stitches by which the close-fitting electrodes are fixed, the close-fitting electrodes and the elastic electric leads are connected, and the thin film is cut and bonded to a frame shape by laser and then hot-pressed onto four sides of the close-fitting electrodes for further fixation. Meanwhile, the stability of the connection of the close-fitting electrodes with the elastic electric leads can be ensued.

Preferably, to reduce the influence of the motion to the connection of the electric generator, the electric connector is disposed at a position corresponding to the right side of the right crotch of the human body; and each of the elastic electric leads is disposed at the position of the crotch, and extends in the right and left directions and is then connected to two symmetrical close-fitting electrodes. Since the surface deformation at the outer side of the right crotch is smallest during the motion of the human body, the stability of electrical connection can be maintained.

In the present invention, preferably, during the fixation of the close-fitting electrodes, the close-fitting electrodes are fixed in an embroidery manner by an embroidery machine; and, during the fixation of the elastic electric leads, the elastic electric leads are fixed in a taping embroidered manner by the embroidery machine.

Preferably, to be convenient for a user to put on the training garment, a tape is disposed on the slide fastener.

In the step of fabric tailoring, preferably, the cloth piece comprises a front chest piece that is located in the middle portion, a first back piece and a second back piece that are respectively located on left and right sides of the front chest piece, a first leg piece that is located on the left side of the front chest piece and below the first back piece, a second leg piece that is located on the right side of the front chest piece and below the second back piece, a collar piece that is located above the front chest piece, and shoulder pieces that are respectively located on two sides of the collar piece.

Compared with the prior art, the present invention has the following advantages:
1. By using a cloth piece in a two-dimensional plane as the fixation object of the electrodes, the electrodes can be fixed by a large-frame embroidery machine, so that a large amount of labor is saved and the two-dimensional cloth piece can be conveniently transformed into a stereoscopic structure suitable for a human body.
2. Since the embroidery machine has a taping embroidery head, the fixation of the electrodes and the taping embroidery procedure of the electric leads can be completed at a time, and the optimal alignment can be realized even if the cloth piece is formed from high-elasticity fabric.
3. Using the compound electrodes and adhering the thin film onto the silver-plated cloth can cause the electric conductivity to be uniform, sweat to be quickly absorbed, and the loss of silver to be reduced. Accordingly, the resistivity can be decreased, and the washing durability can be improved. By compounding the elastic layer and the base cloth, the comfortable fitting of the electrodes to the human body can be realized, and the electrode cloth can be protected as a whole.
4. By using the elastic electric leads, the energy consumption is low, the requirements on the battery life of a portable device can be satisfied, and low loss of silver and good conductivity are still realized after multiple times of washing.

### Brief Description of the Drawings

Fig. 1 is a perspective view of electrodes of a method for manufacturing training garments for use in EMS according to an embodiment of the present invention;
Fig. 2 is a plan view of a semi-finished training garment of the method according to the embodiment of the present invention;
Fig. 3 is a plan view of sleeve pieces of the training garment of the method according to the embodiment of the present invention;
Fig. 4 is a front view of the training garment of the method according to the embodiment of the present invention; and
Fig. 5 is a rear view of the training garment of the method according to the embodiment of the present invention.

### Detailed Description of the Preferred Embodiment

To enable a further understanding of the present invention content of the invention herein, refer to the detailed description of the invention and the accompanying drawings below:
A method for manufacturing training garments for use in EMS, comprises the following steps.

### 1) Preparation of close-fitting electrodes 1, comprising:

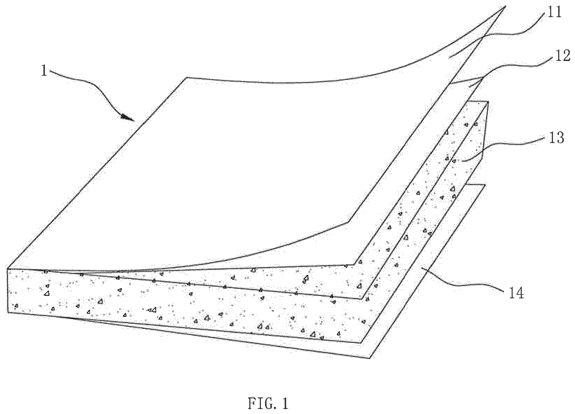

As shown in Fig. 1, each of the electrodes comprises a piece of silver-plated cloth 11, a thin film 12, an elastic layer 13 and a piece of base cloth 14, which are compounded in turn to form the electrode. The base cloth 14 is used for being connected and fixed (generally by sewing) on an inner side of the garment, and the silver-plated cloth 11 is fitted to the muscles. The electrodes are manufactured in a method comprising the following steps:
(i) manufacturing the silver-plated cloth 11, comprising: in the present invention, elastic fabric (warp-knitted fabric, weft-knitted fabric or woven fabric) is woven from nylon fiber and polyurethane. Preferably, the nylon fiber is fine fiber. Chemical silver plating (elargol coating) is performed on the elastic fabric to obtain the silver-plated cloth 11. The surface chemical silver plating technology for nylon fiber is already well known, by which the fabric can be ensured to compact enough, and there is enough physical contact between coils for the purpose of energization. The silver-plated cloth is characterized in that: filaments can be twisted to ensure better elasticity since filaments are fine and have certain electricity; the loss of silver is low at normal temperature; the silver-plated cloth is durable and affine for the skin; and, the released silver ions have very strong bactericidal effect. Alternatively, the silver-plated cloth 11 can also be manufactured by a method described in the background art, in which silver is first plated on the surface of nylon fiber, the nylon fiber is then woven to form elastic fabric, and silver may be plated onto the elastic fabric again.
(ii) adhering the thin film 12, comprising: the thin film 12 is adhered onto the silver-plated cloth 11 by dot-coating. The thin film 12 can improve the absorption of sweat and allow sweat to quickly spread on the surface so that the impedance is reduced quicker after the skin excretes sweat. The thin film realizes better user experience in combination with the electric generator, and can solidify the coils of the silver-plated cloth 11, so that the loss of silver during washing is greatly reduced, the excellent physical contact between the coils can still be maintained after multiple times of washing, and the washing durability is improved.
(iii) compounding the elastic layer 13, comprising: the elastic layer 13 is compounded onto the thin film 12 by flame to obtain compounded electrode cloth. The elastic layer 13 can realize better fitting of the electrodes to the skin of the human body. The elastic layer is preferably sponge having a thickness of 2.0 mm to 8 mm, depending upon the actual requirements. Alternatively, the elastic layer may also be an air layer, cellular fabric or the like.
(iv) compounding the base cloth 14, comprising: in order to reduce the noise caused by the frication with the skin when a person wears the garment, in the electrode cloth, an elastic fabric layer can be compounded on the elastic layer 13 by dot-coating or by flame, as a piece of base cloth 14 for purpose of protection.
(v) cutting, comprising: the electrode cloth obtained in the step (iii) or the step (iv) is cut according to the desired size to serve as the electrodes of the training garment. The obtained close-fitting electrodes have the characteristics of low resistivity, washing durability, uniform electrical conductivity, comfortable fitting effect and quick sweat absorption.

Optionally, according to different requirements, a piece of mesh cloth can be adhered or compounded between the thin film 12 and the base cloth 14.

2) Preparation of elastic electric leads 2, comprising: similarly to the preparation of the silver-plated cloth 1 in the step 1), in the present invention, the electric leads are formed by performing chemical silver plating on nylon fiber to form silver-plated nylon fiber and then twisting and merging a plurality of silver-plated nylon fiber filaments and polyurethane. At present, the longest wiring having length resistivity of 18 to 20 Ω/m, good consistency and maximum size is generally within 1.10 m. Therefore, all leads can be controlled below 30 S2, have low energy consumption and can satisfy the requirements on the battery life of a portable device. Experiments indicate that the loss of silver is low after multiple times of washing, and the performance is maintained well.

The close-fitting electrodes 1 and the elastic electric leads 2 are preferred solutions of the present invention, and the following steps can also be completed by using the electrodes and electric leads in the prior art, as described in CN101536903A in the background art.

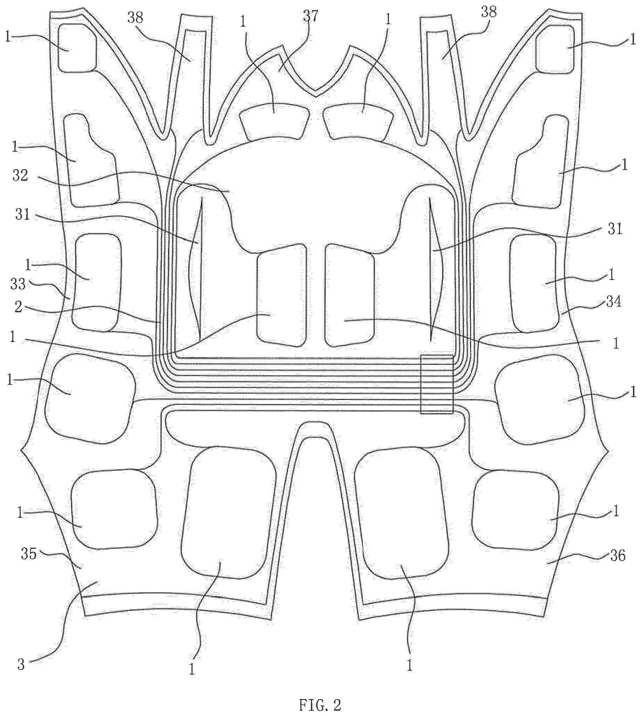

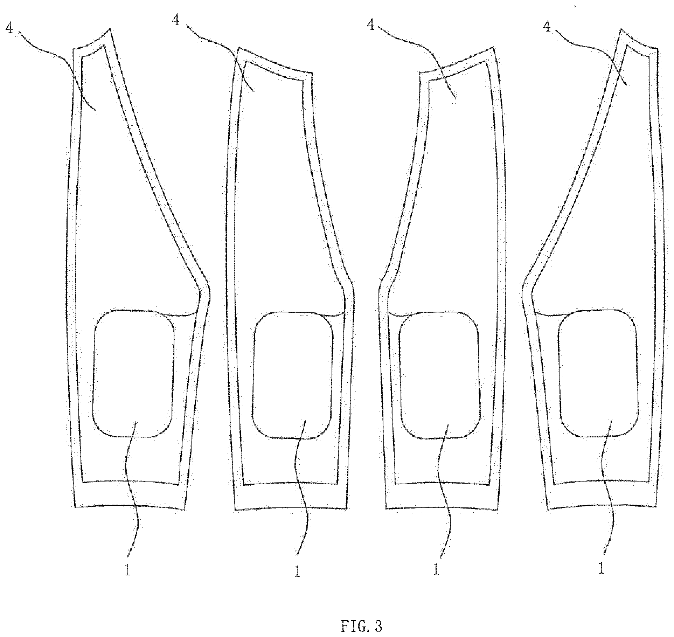

3) Preparing a semi-finished training garment, comprising:
(A) fabric tailoring, comprising: the elastic fabric is tailored into a plane cloth piece 3 shown in Fig. 2 and four plane lower sleeve pieces 4 shown in Fig. 3. The elastic fabric is optionally existing fabric used for common training garments. The cloth piece 3 has a shape which is the same as the shape that is formed by cutting off and then unfolding the middle of the back and the inner side of legs of a common training garment (not comprising sleeves). This shape can be obtained by multiple experiments, so that the finally sewed shape can be fitted to a user's body to the greatest extent. The cloth piece 3 comprises a front chest piece 32 that is located in the middle portion, a first back piece 33 and a second back piece 34 that are respectively located on left and right sides of the front chest piece 32, a first leg piece 35 that is located on the left side of the front chest piece 32 and below the first back piece 33, a second leg piece 36 that is located on the right side of the front chest piece 32 and below the second back piece 34, a collar piece 37 that is located above the front chest piece 32, and shoulder pieces 38 that are respectively located on two sides of the collar piece 37. The first back piece 33 and the second back piece 34 can be spliced to form a complete back piece. There are two groups of lower sleeve pieces 4, and two lower sleeve pieces 4 in each group can be sewed to form a sleeve.
(B) fixing close-fitting electrodes 1, comprising: the close-fitting electrodes 1 obtained in the step 1) are fixed onto a back side of the cloth piece 3 and the lower sleeve pieces 4 in an embroidery manner. The close-fitting electrodes 1 need to directly face and be fitted at positions corresponding to muscle groups of a human body. During the fixation, automatic sewing can be realized by a customized large-frame embroidery machine configured with an ordinary embroidery head, and the ordinary embroidery machine are used for assisting in fixation of the close-fitting electrodes 1 on the lower sleeve pieces 4.
(C) fixing elastic electric leads 2, comprising: the elastic electric leads 2 obtained in the step 2) are also positioned, sewed and fixed on the cloth piece 3 by a customized large-frame embroidery machine with a taping embroidery machine head, and the ordinary embroidery machine is used for assisting in fixation of the elastic electric leads 2 on the lower sleeve pieces 4, so that symmetrical close-fitting electrodes 1 corresponding to the left and right symmetrical muscle groups are connected, as shown in Fig. 2. Subsequently, the elastic electric leads 2 are drawn, by a crochet hook, triangular stitches by which the close-fitting electrodes 1 are fixed, the close-fitting electrodes 1 and the elastic electric leads 2 are connected, and the thin film is cut and bonded to form a frame shape by laser and then hot-pressed onto four sides of the close-fitting electrodes 1 for further fixation. Meanwhile, the stability of the connection of the close-fitting electrodes 1 with the elastic electric leads 2 can be ensued.
(D) sewing waist darts, comprising: synchronously threading at positions of the front chest piece 32 of the cloth piece 3 corresponding to the waistline positions of the human body (the two positions are symmetrical) to define regions 31 for sewing waist darts, and then fixing and reinforcing by sewing densely and zigzagging.
(E) providing an electric generator connector, comprising: since the close-fitting electrodes 1 are distributed over the whole body, an external EMS electric generator is fixed on an outer side of a right crotch where minimum surface deformation will be caused during the motion of the human body. Therefore, an electric connector 5 is provided at this position (located on the front side of the cloth piece 3). The electric connector 5 is connected to all the elastic electric leads 2. Therefore, preferably, each of the elastic electric leads 2 is disposed at the position of the crotch, and extends in the right and left directions and is then connected to two symmetrical close-fitting electrodes 1; and, the electric connector 5 is connected to the external EMS electric generator by a magnetic clasp. Since the paths of the elastic electric leads 2 are distributed on the whole body, the effect of three-dimensional distribution is realized. Moreover, by measuring the total resistance of the circuit passing through the body and by inferring the contact rate, the voltage and current under which the pulse should be released by the EMS electric generator can be calculated.

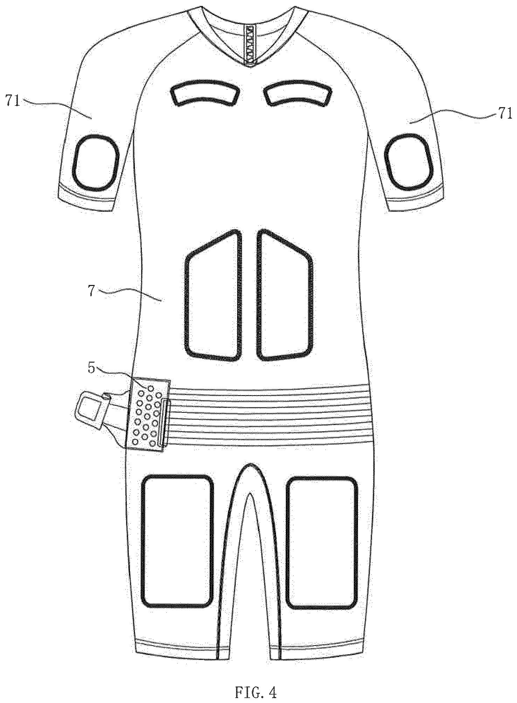

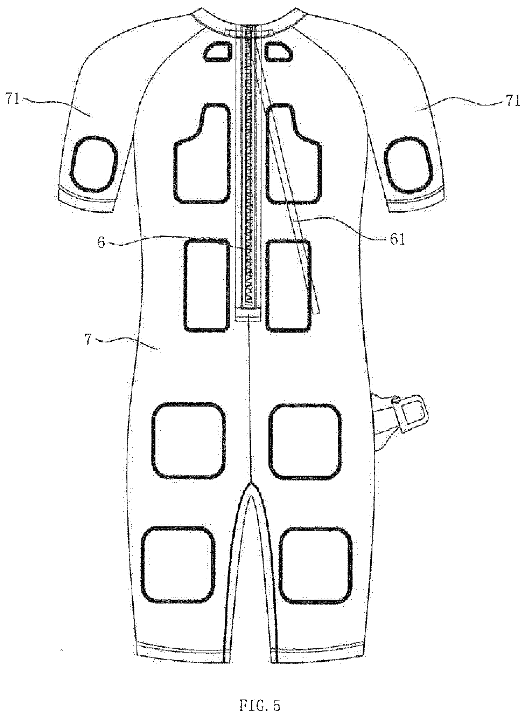

4) Manufacturing a finished training garment, comprising: referring to Fig. 4 and Fig. 5, folding left and right portions of the cloth piece 3 obtained in step E, partially sewing the folded cloth piece 3 at a junction of the inner side of the legs and a junction of the back, providing a slide fastener 6 in the remaining portion of the junction of the back so as to form a garment body 7, sewing the lower sleeve pieces 4 to positions of the garment body 7 corresponding to the arms to form sleeves 71, and connecting the external EMS electric generator to the electric connector 5 to form a training garment for use in EMS. A tape 61 is provided on the slide fastener 6, and the tape 61 is coordinated with the slide fastener 6 so that the user can independently put on the training garment.

The whole cloth piece 3 is designed to a stereoscopic garment in accordance with the whole curve of the human body by providing darts at the waist, sewing at the back, sewing the shoulders and sleeves, providing the collar and the like. It is ensured that each part of the garment (particularly the neck and back that are difficult in fitting) can also be closely fitted to the human body.

The training garment manufactured by the method of the present invention is high in production efficiency and accurate in electrode positioning, and can ensure the fully fitting of the electrodes with the skin at each part of the human body. Unlike the prior art, it is not required to coat gel, hyaluronic acid or the like on the surface of the skin to allow water to penetrate into dermis from epidermis, as long as there is little sweat on the surface of the skin. When there is sweat on the surface of the skin, the resistance between the surface of the skin and the sweat gland is reduced, so that the electrical conductivity of the skin is increased. In this case, when the EMS electric generator applies a low-voltage electrical signal to the skin from the outside, the sweat gland and its internal muscular tissue form an electrical loop. This low-voltage electrical signal has a specific frequency and a proper current value. After this low-voltage electrical signal penetrates into muscles through the skin, the muscles will make a response, similar to that made under the control of the brain nervous system.

## Claims

1. A method for manufacturing training garments for use in Electrical Muscle Stimulation (EMS), comprising the following steps:
1) preparing a semi-finished training garment, comprising:
(A) fabric tailoring, comprising: tailoring a piece of elastic fabric to a plane cloth piece (3) and lower sleeve pieces (4), the plane cloth piece having: left and right portions, a front chest piece (32), a first back piece (33) and a second back piece (34), the front chest piece located in a middle portion of the plane cloth piece and the first and second back pieces located on respective left and right sides of the front chest piece; a first leg piece (35) located on the left side of the front chest piece and below the first back piece, and a second leg piece (36) located on the right side of the front chest piece and below the second back piece; a collar piece (37) located above the front chest piece; and shoulder pieces (38) that are located on respective sides of the collar piece;
(B) fixing close-fitting electrodes (1), comprising: fixing a plurality of close-fitting electrodes (1) onto a back side of the cloth piece (3) and lower sleeve pieces (4) by an embroidery machine, in which the close-fitting electrodes (1), in use, directly face and are fitted at positions corresponding to muscle groups of a human body, wherein each of the close-fitting electrodes (1) comprises a piece of silver-plated cloth (11), a thin film (12) and an elastic layer (13), which are compounded in turn;
(C) fixing elastic electric leads (2), comprising: fixing elastic electric leads (2) onto the back side of the cloth piece (3) and lower sleeve pieces (4) by the embroidery machine, so as to connect symmetrical close-fitting electrodes (1) together;
(D) sewing waist darts, comprising: synchronously threading at positions of the cloth piece (3) corresponding to the waistline positions of the human body to define regions (31) for sewing waist darts, and then fixing and reinforcing by sewing densely and zigzagging;
(E) providing an external EMS electric generator and an electric generator connector, comprising: providing an electric connector (5) on a front side of the cloth piece (3), the electric connector (5) configured for being connected to said external EMS electric generator, the electric connector (5) being electrically connected to all the elastic electric leads (2) on the back side of the cloth piece (3); and
2) manufacturing a finished training garment, comprising:
folding left and right portions of the cloth piece (3) obtained in step (E), partially sewing the folded cloth piece (3) at a junction of inner sides of each of the leg pieces and at a junction of the first and second back pieces, providing a slide fastener (6) in the remaining portion of the junction of the back pieces so as to form a garment body (7), sewing the lower sleeve pieces (4) to positions of the garment body (7) corresponding to the arms to form sleeves (71), and connecting the external EMS electric generator to the electric connector (5) to form a training garment for use in EMS.

2. The method according to claim 1, in which the close-fitting electrodes (1) are manufactured in a method comprising the following steps:
(i) manufacturing the silver-plated cloth (11), comprising: using nylon fiber as a substrate to obtain the silver-plated cloth (11);
(ii) adhering the thin film (12), comprising: adhering the thin film (12) onto the silver-plated cloth (11);
(iii) compounding the elastic layer (13), comprising: compounding the elastic layer (13) on the thin film (12) to obtain the compounded electrode cloth; and
(iv) cutting, comprising: cutting the electrode cloth according to the desired size to obtain the close-fitting electrodes (1).

3. The method according to claim 2, in which, in step (iii) of manufacturing the close-fitting electrodes (1), an elastic fabric layer is further compounded on the elastic layer (13) of the electrode cloth to form a protective base cloth (14).

4. The method according to claim 1, in which the elastic electric leads (2) are formed by twisting and merging a plurality of silver-plated nylon fiber filaments and polyurethane.

5. The method according to claim 1, in which, in the step of fixing the elastic electric leads (2), the elastic electric leads (2) are drawn, by a crochet hook, into triangular stitches by which the close-fitting electrodes (1) are fixed, the close-fitting electrodes (1) and the elastic electric leads (2) are connected, and the thin film is cut and bonded to a frame shape by a laser and then hot-pressed onto four sides of the close-fitting electrodes (1) for further fixation.

6. The method according to any one of claim 1-5, in which the electric connector (5) is disposed at a position corresponding to the right side of the right crotch of the human body;and
each of the elastic electric leads (2) is disposed at the position of the crotch, and extends in right and left directions and is then connected to two symmetrical close-fitting electrodes (1).

7. The method according to any one of claim 1-5, in which, during the fixation of the close-fitting electrodes (1), the close-fitting electrodes (1) are fixed in an embroidered manner by an embroidery machine.

8. The method according to any one of claim 1-5, in which, during the fixation of the elastic electric leads (2), the elastic electric leads (2) are fixed in a taping embroidered manner by the embroidery machine.

9. The method according to anyone of claim 1-5, in which a tape (61) is disposed on the slide fastener (6).

## Patentansprüche

1. Ein Verfahren zur Herstellung von Trainingsbekleidung zur Verwendung bei elektrischer Muskelstimulation (EMS), beinhaltend die folgenden Schritte:
1) Anfertigen eines halbfertigen Trainingskleidungsstücks, beinhaltend:
(A) Zuschneiden einer Textilie, beinhaltend: Zuschneiden eines Stücks elastischer Textilie zu einem ebenen Stück Stoff (3) und unteren Ärmelteilen (4), wobei das ebene Stück Stoff Folgendes aufweist: einen linken und einen rechten Abschnitt, ein vorderes Brustteil (32), ein erstes Rückenteil (33) und ein zweites Rückenteil (34), wobei sich das vordere Brustteil in einem mittleren Abschnitt des ebenen Stücks Stoff befindet und sich das erste und das zweite Rückenteil auf jeweils der linken bzw. rechten Seite des vorderen Brustteils befinden; ein erstes Beinteil (35), das sich auf der linken Seite des vorderen Brustteils und unterhalb des ersten Rückenteils befindet, und ein zweites Beinteil (36), das sich auf der rechten Seite des vorderen Brustteils und unterhalb des zweiten Rückenteils befindet; ein Kragenteil (37), das sich oberhalb des vorderen Brustteils befindet; und Schulterteile (38), die sich auf jeweiligen Seiten des Kragenteils befinden;
(B) Befestigen von eng anliegenden Elektroden (1), beinhaltend: Befestigen einer Vielzahl von eng anliegenden Elektroden (1) auf einer Rückseite des Stücks Stoff (3) und der unteren Ärmelteile (4) durch eine Stickmaschine, wobei die eng anliegenden Elektroden (1) bei Verwendung Muskelgruppen eines menschlichen Körpers direkt gegenüberliegen und an Positionen angebracht sind, die diesen entsprechen, wobei jede der eng anliegenden Elektroden (1) ein Stück versilberten Stoffs (11), eine dünne Folie (12) und eine elastische Schicht (13), die der Reihe nach zusammengefügt sind, beinhaltet;
(C) Befestigen von elastischen elektrischen Leitungen (2), beinhaltend: Befestigen von elastischen elektrischen Leitungen (2) auf der Rückseite des Stücks Stoff (3) und der unteren Ärmelteile (4) durch die Stickmaschine, um symmetrische eng anliegende Elektroden (1) miteinander zu verbinden;
(D) Nähen von Taillenabnähern, beinhaltend: synchrones Abstecken an Positionen des Stücks Stoff (3), die den Taillenpositionen des menschlichen Körpers entsprechen, um Bereiche (31) zum Nähen von Taillenabnähern zu definieren, und dann Befestigen und Verstärken durch dichtes Nähen im Zickzack;
(E) Bereitstellen eines externen EMS-Elektrogenerators und eines Elektrogeneratorverbinders, beinhaltend: Bereitstellen eines elektrischen Verbinders (5) auf einer Vorderseite des Stücks Stoff (3), wobei der elektrische Verbinder (5) konfiguriert ist, um mit dem externen EMS-Elektrogenerator verbunden zu sein, wobei der elektrische Verbinder (5) mit allen elastischen elektrischen Leitungen (2) auf der Rückseite des Stücks Stoff (3) elektrisch verbunden ist; und
2) Herstellen eines fertigen Trainingskleidungsstücks, beinhaltend:
Falten eines linken und rechten Abschnitts des Stücks Stoff (3), das in Schritt (E) erhalten wurde, teilweises Vernähen des gefalteten Stücks Stoff (3) an einer Verbindungsstelle zwischen Innenseiten jedes der Beinteile und an einer Verbindungsstelle zwischen dem ersten und zweiten Rückenteil, Bereitstellen eines Reißverschlusses (6) in dem verbleibenden Abschnitt der Verbindungsstelle der Rückenteile, um einen Kleidungsstückkörper (7) zu bilden, Annähen der unteren Ärmelteile (4) an Positionen des Kleidungsstückkörpers (7), die den Armen entsprechen, um Ärmel (71) zu bilden, und Verbinden des externen EMS-Elektrogenerators mit dem elektrischen Verbinder (5), um ein Trainingskleidungsstück zur EMS-Verwendung zu bilden.

2. Verfahren gemäß Anspruch 1, wobei die eng anliegenden Elektroden (1) in einem Verfahren hergestellt werden, das die folgenden Schritte beinhaltet:
(i) Herstellen des versilberten Stoffs (11), beinhaltend: Verwenden von Nylonfaser als ein Substrat, um den versilberten Stoff (11) zu erhalten;
(ii) Aufkleben der dünnen Folie (12), beinhaltend: Aufkleben der dünnen Folie (12) auf den versilberten Stoff (11);
(iii) Anfügen der elastischen Schicht (13), beinhaltend: Anfügen der elastischen Schicht (13) an die dünne Folie (12), um den zusammengefügten Elektrodenstoff zu erhalten; und
(iv) Schneiden, beinhaltend: Schneiden des Elektrodenstoffs gemäß der gewünschten Größe, um die eng anliegenden Elektroden (1) zu erhalten.

3. Verfahren gemäß Anspruch 2, wobei in Schritt (iii) des Herstellens der eng anliegenden Elektroden (1) ferner eine elastische Textilschicht an die elastische Schicht (13) des Elektrodenstoffs angefügt wird, um einen schützenden Basisstoff (14) zu bilden.

4. Verfahren gemäß Anspruch 1, wobei die elastischen elektrischen Leitungen (2) durch das Verdrehen und Zusammenführen einer Vielzahl von versilberten Nylonfaserfilamenten und Polyurethan gebildet werden.

5. Verfahren gemäß Anspruch 1, wobei in dem Schritt des Befestigens der elastischen elektrischen Leitungen (2) die elastischen elektrischen Leitungen (2) durch eine Häkelnadel in dreieckige Maschen gezogen werden, wodurch die eng anliegenden Elektroden (1) befestigt werden, die eng anliegenden Elektroden (1) und die elastischen elektrischen Leitungen (2) verbunden werden und die dünne Folie durch einen Laser auf eine Rahmenform geschnitten und gebondet wird und dann zur weiteren Befestigung auf vier Seiten der eng anliegenden Elektroden (1) heiß aufgepresst wird.

6. Verfahren gemäß einem der Ansprüche 1-5, wobei der elektrische Verbinder (5) an einer Position angeordnet wird, die der rechten Seite der rechten Leistenregion des menschlichen Körpers entspricht; und
jede der elastischen elektrischen Leitungen (2) an der Position der Leistenregion angeordnet wird und sich in rechter und linker Richtung erstreckt und dann mit zwei symmetrischen eng anliegenden Elektroden (1) verbunden wird.

7. Verfahren gemäß einem der Ansprüche 1-5, wobei während des Befestigens der eng anliegenden Elektroden (1) die eng anliegenden Elektroden (1) mittels Stickerei durch eine Stickmaschine befestigt werden.

8. Verfahren gemäß einem der Ansprüche 1-5, wobei während des Befestigens der eng anliegenden Elektroden (2) die eng anliegenden Elektroden (2) mittels Taping-Stickerei durch die Stickmaschine befestigt werden.

9. Verfahren gemäß einem der Ansprüche 1-5, wobei ein Band (61) auf dem Reißverschluss (6) angeordnet wird.

## Revendications

1. Un procédé pour la fabrication de vêtements d'entraînement destinés à être utilisés en stimulation musculaire électrique (EMS), comprenant les étapes suivantes :
1) la préparation d'un vêtement d'entraînement semi-fini, comprenant :
(A) le façonnage de tissu, comprenant : le façonnage d'une pièce de tissu élastique en une pièce de textile (3) plane et des pièces de manche inférieures (4), la pièce de textile plane ayant : des portions gauche et droite, une pièce de poitrine avant (32), une première pièce de dos (33) et une deuxième pièce de dos (34), la pièce de poitrine avant étant située dans une portion médiane de la pièce de textile plane et les première et deuxième pièces de dos étant situées sur des côtés gauche et droit respectifs de la pièce de poitrine avant ; une première pièce de jambe (35) située sur le côté gauche de la pièce de poitrine avant et au-dessous de la première pièce de dos, et une deuxième pièce de jambe (36) située sur le côté droit de la pièce de poitrine avant et au-dessous de la deuxième pièce de dos ; une pièce d'encolure (37) située au-dessus de la pièce de poitrine avant ; et des pièces d'épaule (38) qui sont situées sur des côtés respectifs de la pièce d'encolure ;
(B) la fixation d'électrodes à ajustement serré (1), comprenant : la fixation d'une pluralité d'électrodes à ajustement serré (1) à un côté arrière de la pièce de textile (3) et des pièces de manche inférieures (4) par une machine à broder, les électrodes à ajustement serré (1), lors de l'utilisation, faisant directement face et étant ajustées à des positions correspondant à des groupes musculaires d'un corps humain, chacune des électrodes à ajustement serré (1) comprenant une pièce de textile argenté (11), un film mince (12) et une couche élastique (13), qui sont combinés tour à tour ;
(C) la fixation de conducteurs électriques élastiques (2), comprenant : la fixation de conducteurs électriques élastiques (2) au côté arrière de la pièce de textile (3) et des pièces de manche inférieures (4) par la machine à broder, de façon à connecter ensemble des électrodes à ajustement serré (1) symétriques ;
(D) la couture de pinces de taille, comprenant : le faufilage synchrone au niveau de positions de la pièce de textile (3) correspondant aux positions du tour de taille du corps humain pour définir des zones (31) pour la couture de pinces de taille, puis la fixation et le renforcement par couture de façon dense et en zigzag ;
(E) la fourniture d'un générateur électrique EMS externe et d'un connecteur de générateur électrique, comprenant : la fourniture d'un connecteur électrique (5) sur un côté avant de la pièce de textile (3), le connecteur électrique (5) étant configuré pour être connecté audit générateur électrique EMS externe, le connecteur électrique (5) étant connecté électriquement à tous les conducteurs électriques élastiques (2) sur le côté arrière de la pièce de textile (3) ; et
2) la fabrication d'un vêtement d'entraînement fini, comprenant :
le pliage de portions gauche et droite de la pièce de textile (3) obtenue à l'étape (E), la couture partielle de la pièce de textile (3) pliée au niveau d'une jonction de côtés internes de chacune des pièces de jambe et au niveau d'une jonction des première et deuxième pièces de dos, la fourniture d'une fermeture à glissière (6) dans la portion restante de la jonction des pièces de dos de façon à former un corps de vêtement (7), la couture des pièces de manche inférieures (4) à des positions du corps de vêtement (7) correspondant aux bras pour former des manches (71), et la connexion du générateur électrique EMS externe au connecteur électrique (5) pour former un vêtement d'entraînement destiné à être utilisé en EMS.

2. Le procédé selon la revendication 1, dans lequel les électrodes à ajustement serré (1) sont fabriquées selon un procédé comprenant les étapes suivantes :
(i) la fabrication du textile argenté (11), comprenant : l'utilisation de fibre de nylon comme substrat pour obtenir le textile argenté (11) ;
(ii) la réalisation de l'adhérence du film mince (12), comprenant : la réalisation de l'adhérence du film mince (12) au textile argenté (11) ;
(iii) la combinaison de la couche élastique (13), comprenant : la combinaison de la couche élastique (13) sur le film mince (12) pour obtenir le textile d'électrode combiné ; et
(iv) la coupe, comprenant : la coupe du textile d'électrode selon les dimensions souhaitées pour obtenir les électrodes à ajustement serré (1).

3. Le procédé selon la revendication 2, dans lequel, à l'étape (iii) de fabrication des électrodes à ajustement serré (1), une couche de tissu élastique est en outre combinée sur la couche élastique (13) du textile d'électrode pour former un textile de base protecteur (14).

4. Le procédé selon la revendication 1, dans lequel les conducteurs électriques élastiques (2) sont formés par torsion et fusion d'une pluralité de filaments de fibre de nylon argentée et de polyuréthane.

5. Le procédé selon la revendication 1, dans lequel, à l'étape de fixation des conducteurs électriques élastiques (2), les conducteurs électriques élastiques (2) sont tirés, par une aiguille à crochet, dans des points de liage triangulaires par lesquels les électrodes à ajustement serré (1) sont fixées, les électrodes à ajustement serré (1) et les conducteurs électriques élastiques (2) sont connectés, et le film mince est coupé et assemblé à une forme de cadre par un laser puis pressé à chaud sur quatre côtés des électrodes à ajustement serré (1) pour une fixation supplémentaire.

6. Le procédé selon l'une quelconque des revendications 1 à 5, dans lequel le connecteur électrique (5) est disposé à une position correspondant au côté droit de l'entrejambe droit du corps humain ; et
chacun des conducteurs électriques élastiques (2) est disposé à la position de l'entrejambe, et s'étend dans des directions droite et gauche et est ensuite connecté à deux électrodes à ajustement serré (1) symétriques.

7. Le procédé selon l'une quelconque des revendications 1 à 5, dans lequel, pendant la fixation des électrodes à ajustement serré (1), les électrodes à ajustement serré (1) sont fixées d'une manière brodée par une machine à broder.

8. Le procédé selon l'une quelconque des revendications 1 à 5, dans lequel, pendant la fixation des conducteurs électriques élastiques (2), les conducteurs électriques élastiques (2) sont fixés d'une manière brodée en bande par la machine à broder.

9. Le procédé selon l'une quelconque des revendications 1 à 5, dans lequel une bande (61) est disposée sur la fermeture à glissière (6).
